(19) Europäisches Patentamt
European Patent Office
Office européen des brevets

(11) Publication number: 0 369 497 A1

(12) **EUROPEAN PATENT APPLICATION**

(21) Application number: 89202414.2

(22) Date of filing: 26.09.89

(51) Int. Cl.[5]: C07F 9/58, C07F 9/38, C07C 229/16, A61K 49/00, C07D 257/02, C07D 273/00

(30) Priority: 27.09.88 US 249744

(43) Date of publication of application: 23.05.90 Bulletin 90/21

(84) Designated Contracting States: ES GR

(71) Applicant: SALUTAR, INC.
428 Oakmead Parkway
Sunnyvale California 94086(US)

(72) Inventor: Rocklage, Scott
20221 La Paloma Avenue
Saratoga California 95070(US)
Inventor: Cacheris, William
999 Evelyn Terrace West No. 9
Sunnyvale California 94086(US)
Inventor: Jamieson, Gene
18155 China Grade Road
Boulder Creek California 95006(US)

(74) Representative: Cockbain, Julian et al
Frank B. Dehn & Co. Imperial House 15-19, Kingsway
London WC2B 6UZ(GB)

(54) **Manganese (II) chelates.**

(57) A process is provided for the preparation of Mn(II) chelates for use as contrast agents in magnetic resonance imaging, said process comprising reacting insoluble manganese (II) oxide with an aqueous suspension comprising a molar equivalent or molar excess of an insoluble protonated chelating compound conveniently at a temperature of from 20 to 50 °C. When the reaction is carried out with a protonated chelating agent in the absence of base, a precipitate of the protonated Mn(II) chelate is formed. A low osmolarity Mn(II) chelate solution can be formed from the precipitates by dissolving them in an aqueous solution of base. When the initial chelate forming reaction is carried out in a solution containing a molar equivalent or excess of base, a low osmolarity solution of the Mn(II) chelate is directly formed with most chelating agents. The Mn(II) chelate precipitates and low osmolarity solutions formed by the above processes are also aspects of this invention.

EP 0 369 497 A1

# MANGANESE(II) CHELATES

This invention relates to novel protonated manganese(II) chelates, to low osmolarity magnetic resonance imaging (MRI) contrast media containing or prepared with them and to methods for their preparation.

In diagnostic imaging, contrast agents are frequently used to enhance the image contrast, for example between different organs or between healthy and unhealthy tissue within the same organ. The nature and manner of operation of the contrast agents depend upon the nature of the imaging technique and the organ or tissue which is to be imaged.

Thus in magnetic resonance imaging (MRI), image contrast may be enhanced by introducing into the body zone being imaged a contrast agent which affects the nuclear spin reequilibration characteristics of the nuclei (generally water protons in body tissues or fluids) which are responsible for the magnetic resonance (MR) signals from which the MR images are generated.

In 1978 Lauterbur (see Lauterbur et al., pages 752-759 in "Electrons to Tissues - Frontiers of Biological Energetics", Vol. 1, edited by Dutton et al., Academic Press, NY, 1978) proposed the use of paramagnetic metal ions, such as Mn(II), as MRI contrast agents.

Mn(II) chelates are particularly useful as MRI contrast agents because manganese, being normally present in the body in low concentrations, is less toxic than many paramagnetic metal ions, and because Mn(II) is particularly effective at enhancing MRI contrast. A number of Mn(II) chelates have been investigated for use as MRI contrast agents and are reported in the literature.

Chelates of paramagnetic metal ions are normally formed by reacting an aqueous solution of a paramagnetic metal salt with the chelating agent in approximately equimolar proportions to yield an acidic solution of the chelate and salt anion. An alkaline agent such as sodium hydroxide is added to neutralize the solution to a physiologically acceptable pH, introducing further ions (cations).

Thus for example US-A-4647447 (Schering AG) describes the preparation of Mn(II) chelates of diethylenetriaminepentaacetic acid (DTPA), trans-1,2-diamino-cyclohexane-N,N,N′,N′-tetraacetic acid (DCTA), ethylenediaminetetraacetic acid (EDTA), and a variety of other chelating agents. Also disclosed is the preparation of paramagnetic metal ion chelates of ethylenedinitrilotetrakis(methylphosphonic acid) (EDTP) and 1,4,7,10-tetraazacyclododecanetetraacetic acid (DOTA). The methods described in this patent for preparing Mn(II) chelates comprise the reaction of $MnCO_3$, a water-soluble manganese salt, with the chelating agent in aqueous solution to yield a chelate solution containing the carbonate ion. Sodium hydroxide is added to raise the pH to 7. The solution is then generally evaporated to dryness. In Example 13 of US-A-4647447 $MnCO_3$ is reacted with ethylenedinitrilotetra(acetohydroxamic acid), acetone is added, and after several hours, a precipitated crystallizate of Mn(II) complex is removed, washed and dried at 50° C in vacuo to yield the dihydrate. In Example 52, $MnCO_3$ is reacted with DTPA, and the solution is heated to 95° C, neutralized with sodium hydroxide solution, filtered and put into ampoules. The preparation of a lipid conjugate and its purification using a Sephadex G50 gel filtration column is also described in Example 57 of this patent.

Such processes yield a hyperosmotic injection solution resulting in an increased $LD_{50}$ for the drug product. Substantial efforts would then be required to separate the chelate from the soluble ions, in order to lower the toxicity of the final product formulation.

It may also be noted that where Mn(II) chelates have previously been prepared as part of studies of the chelating tendencies of metal ions, reagent grade nitrates and chlorides have been used. See for example Frost, A. et al, J.Am.Chem.Soc. 80:530 (1958); L'Eplattenier, F. et al, J.Am.Chem.Soc. 89:837 (1967)).

A need exists for improved MRI contrast media and it is an objective of the present invention to develop novel Mn(II) chelates suitable for the preparation of low osmolarity contrast media.

The present invention is based on the surprising discovery that a Mn(II) chelate may be formed by reacting an essentially water insoluble manganese compound, manganese(II) mono-oxide (MnO), with an essentially water-insoluble form of the chelating agent; it had previously been thought that a soluble form of Mn(II) (i.e. a salt) was required for chelation, thereby necessitating the introduction of undesired counteranions.

Thus in one aspect, the present invention provides a substantially protonated, water-insoluble Mn(II) chelate.

The term "substantially protonated" as used herein indicates that protons represent at least 75% of the labile cations in the chelate product or chelating compound. In preferred embodiments, the chelate of chelating compound is fully protonated i.e. protons represent 100% of the cations therein. Most preferably, the protons are acidic protons. It will therefore be realized that the Mn(II) chelate of the invention may be a neutral species or may be in the form of a salt of a chelate ion with a, preferably physiologically tolerable,

counterion.

The chelates of the invention may be formed by reacting MnO with an aqueous suspension comprising a molar equivalent or molar excess of the chelating compound. When the reaction is carried out at a pH of 3 to 5 a fully protonated Mn(II) chelate precipitates. This precipitated, fully protonated chelate may if desired be converted into a soluble salt form, e.g. by reaction with a base for example to remove one or more labile acid protons.

Thus, in a further aspect the invention provides a process for the preparation of a Mn(II) chelate, said process comprising reacting MnO with a substantially-protonated, water-insoluble chelating compound in an aqueous medium, e.g. water, and if desired subsequently dissolving the precipitated Mn(II) chelate in an aqueous, preferably physiologically tolerable, base solution.

If however, the chelation reaction is carried out in a solution containing a molar equivalent or excess of an inorganic or organic base (preferably a physiologically tolerable base), a low osmolarity solution of the chelate is prepared directly. Thus these processes can yield the desired products directly, without the need for elaborate and costly purification procedures to remove excess ions.

MRI contrast media can be formulated from the solid chelates by dissolving the precipitated chelate in an aqueous solution containing 1 to 6, preferably 1 to 3, molar equivalents of pharmaceutically acceptable, non-toxic inorganic and/or organic base. Suitable bases include those having as cations lithium, sodium and especially calcium ions. Suitable organic bases include ethanolamine, diethanolamine, morpholine, glucamine, N,N-dimethylglucamine and N-methylglucamine, for example. The solutions can be formulated at any temperature, but at temperatures of 20 to 40 °C, the dissolution rates are most efficient.

The essential step in this process of the invention is the reaction of MnO, initially in a particulate form, with an aqueous suspension of the chelating compound. The chelating compound is conveniently present, preferably in a fully protonated or free acid form, in equimolar proportions (1:1) or a molar excess to ensure solubilization of all of the MnO. Preferably, the MnO and chelating compound are reacted in equimolar proportions. The reaction is conveniently carried out at a temperature of from 20 to 50 °C, and preferably from 20 to 30 °C. The reaction proceeds more rapidly at the elevated temperatures.

In one preferred embodiment of the process of the invention the reaction is carried out in the absence of base at a pH of not more than 5 e.g. 3-5, or even less than 3, and a precipitate is formed which can easily be removed from the solution. A reaction time of from 8 to 10 hours is usually sufficient for completion of the reaction at temperatures above 20 °C, and the fully protonated Mn(II) chelate product precipitates immediately from the acidic solution. It can be separated from the reaction mixture, washed, dried, and stored for later preparation of the MRI contrast media.

Alternatively, for chelating compounds other than EDTA, the reaction may conveniently be carried out in the presence of the 1-6 molar equivalents amount of base. Preferably, the chelating compound is dissolved in the base solution prior to the addition of the insoluble MnO. A reaction time of from 8 to 10 hours is usually sufficient for completion of the reaction at temperatures above 20 °C. This process directly yields a low osmolarity Mn(II) chelate solution suitable for use as an MRI contrast medium after appropriate dilution, and may be carried out using most chelating compounds other than EDTA. The resulting solution can be filtered, sterilized, and distributed to sterile containers immediately.

Any chelating compound which forms a stable chelate with the Mn(II) ion may be used in the process of the invention, although those having acidic protons are preferred. Suitable compounds include those of Formula I,

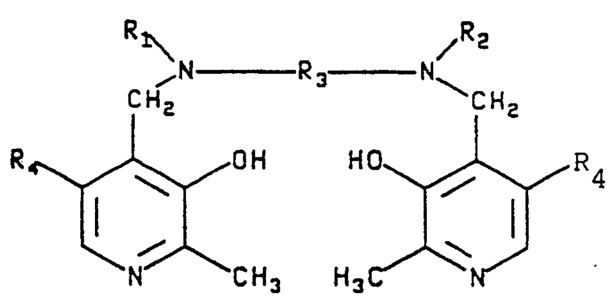

(I)

(wherein
$R_1$ represents a hydrogen atom or a group
$-CH_2-\overset{O}{\overset{\|}{C}}-R_5$, and
$R_2$ represents a hydrogen atom or a group
$-CH_2-\overset{O}{\overset{\|}{C}}-R_6$
with the proviso that one of the groups $R_1$ and $R_2$ is other than hydrogen;
$R_3$ represents a $C_{1-8}$ alkylene, $C_{5-8}$ 1,2-cycloalkylene, or $C_{6-10}$ 1,2-arylene group;
each of the groups $R_4$ which may be the same or different represents a hydrogen atom, a $C_{1-6}$ alkyl group or a group $-CH_2OH$, or a group
$-CH_2-O-\overset{O}{\overset{\|}{P}}(OH)_2$;
with the proviso that where one $R_4$ represents hydrogen or alkyl the other represents a group $-CH_2OP(O)(OA)_2$;
$R_5$ and $R_6$ which may be the same or different each substituted, alkoxy, amino or $C_{1-8}$ alkylamino group), and pharmaceutically acceptable salts, and where group $R_4$ represents a group $CH_2$-O-PO($OH_2$), the phosphate group mono and diesters thereof with mono and polyhydric $C_{1-18}$ alkyl- or alkylamino alcohols.

Preferred chelating compounds include compounds of Formula I wherein $R_5$ and $R_6$ each individually represent $C_{1-8}$ alkoxy, hydroxyethoxy, dihydroxypropoxy (i.e. residues of ethylene glycol or glycerol respectively), amino or $C_{1-8}$ alkylamino groups, or especially preferably $R_5$ and $R_6$ each represent a hydroxy group and the salts of such compounds.

Compounds of Formula I and processes for their preparation are described in EP-A-290047 (Salutar Inc) and EP-A-292761 (Salutar Inc.) which are hereby incorporated by reference in their entirety.

The term "alkyl" and "alkylene", as used herein, include both straight and branch-chain, unsaturated and, particularly, saturated hydrocarbon structures. The term "1,2-cycloalkylene" includes both cis and trans cycloalkyl groups and alkyl substituted cycloalkylene groups bonded at the 1,2-positions to respective nitrogen atoms and alkyl substituted derivatives thereof having from 3 to 8 carbons. The term "1,2-arylene" includes phenyl, pyridyl and naphthyl groups bonded at the 1,2-positions to respective nitrogen atoms and $C_{3-10}$ alkyl substituted derivatives thereof.

Since not all of the acidic protons of the chelates are substituted by the central paramagnetic ion, the solubility of the chelate can be increased if a number of the remaining protons are converted to salts of the conjugate base with physiologically biocompatible cations of inorganic and/or organic bases or basic amino acids. For example, lithium, sodium and especially calcium ions are suitable inorganic countercations. Suitable organic cations include, for example, those of organic bases such as ethanolamine, diethanolamine, morpholine, glucamine, N,N-dimethylglucamine, N-methylglucamine. Ions of amino acids such as lysine, arginine and orithine are also suitable counter cations as generally are those of other bases or naturally occuring acids.

By way of example suitable chelating compounds of Formula I include
N,N′-bis(pyridoxal-5-phosphate)-1,3-(n-propylene)-N,N′-diacetic acid;
N,N′-bis(pyridoxal-5-phosphate)-1,2-(n-propylene)-N,N′-diacetic acid;
N,N′-bis(pyridoxal-5-phosphate)-1,2-isopropylene-N,N′-diacetic acid;
N,N′-bis(pyridoxal-5-phosphate)-1,2-(n-butylene)-N,N′-diacetic acid;
N,N′bis(pyridoxal-5-phosphate)-1,4-(n-butylene)-N,N′-diacetic acid;
N,N′-bis(pyridoxal-5-phosphate)-1,3-(n-butylene)-N,N′-diacetic acid;
N,N′-bis(pyridoxal-5-phosphate)-1,2-(3-methylene)propyl-N,N′-diacetic acid;
N,N′-bis(pyridoxal-5-phosphate)-trans-1,2-cyclopentylenediamine-N,N′-diacetic acid;
N,N′-bis(pyridoxal-5-phosphate)-trans-1,2-cycloheptylenediamine-N,N′-diacetic acid;
N,N′-bis(pyridoxal-5-phosphate)-trans-1,2-cyclooctylenediamine-N,N′diacetic acid;
N,N′bis(pyridoxal-5-phosphate)-1,2-phenylenediamine-N,N′-diacetic acid;
N,N′-bis(pyridoxal-5-phosphate)-cis-1,2-cyclohexylenediamine-N,N′diacetic acid;
N,N′-bis(pyridoxal-5-phosphate-(N-methylethanolamine)monoester)-ethylenediamine-N,N′-diacetic acid;
N,N′-bis(pyridoxal-5-phosphate(N-methylethanolamine)monoester)-1,3-(n-propylene)diamine- N,N′-diacetic acid;
N,N′-bis(pyridoxal-5-phosphate(N-methylethanolamine)monoester)-1,2-(n-propylene)diamine-N,N′diacetic acid;
N,N′-bis(pyridoxal-5-phosphate(N-methylethanolamine)monoester)-1,2-isopropylenediamine-N,N′diacetic acid;

N,N′-bis(pyridoxal-5-phosphate(N-methylethanolamine)monoester)-1,2-(n-butylene)diamine-N,N′diacetic acid;
N,N′-bis(pyridoxal-5-phosphate(N-methylethyanolamine)monoester)-1,4-(n-butylene)diamine-N,N′-diacetic acid;
N,N′-bis(pyridoxal-5-phosphate(N-methylethanolamine)monoester)-1,3-(n-butylene)diamine-N,N′-diacetic acid;
N,N′-bis(pyridoxal-5-phosphate(N-methylethanolamine)monoester)-1,2,(3-methyl)propylenediamine-N,N′-diacetic acid;
N,N′bis(pyridoxal-5-phosphate(N-methylethanolamine)monoester)-trans-1,2-cyclohexylenediamine-N,N′-diacetic acid;
N,N′-bis(pyridoxal-5-phosphate(N-methylethanolamine)monester)-trans-1,2-cyclopentylenediamine-N,N′-diacetic acid;
N,N′-bis(pyridoxal-5-phosphate(N-methylethanolamine)monoester)-trans-1,2-cycloheptylenediamine-N,N′-diacetic acid;
N,N′-bis(pyridoxal-5-phosphate(N-methylethanolamine)monoester)-trans-1,2-cyclooctylenediamine-N,N′-diacetic acid;
N,N′-bis(pyridoxal-5-phosphate(N-methylethanolamine)monoester)-1,2-phenylenediamine-N,N′-diacetic acid;
N,N′-bis(pyridoxal-5-phosphate(N-methylethanolamine)monoester)-cis-1,2-cyclohexylenediamine-N,N′-diacetic acid;
N,N′-bis(pyridoxal-5-phosphate)-ethylenediamine-N-acetic acid;
N,N′-bis(pyridoxal-5-phosphate)-1,3-(n-propylene)diamine-N-acetic acid;
N,N′-bis(pyridoxal-5-phosphate)-1,2-(n-propylene)diamine-N-acetic acid;
N,N′-bis(pyridoxal-5-phosphate)-1,2-isopropylenediamine-N-acetic acid;
N,N′-bis(pyridoxal-5-phosphate)-1,2-(n-butylene)diamine-N-acetic acid;
N,N′-bis(pyridoxal-5-phosphate)-1,4-(n-butylene)diamine-N-acetic acid;
N,N′-bis(pyridoxal-5-phosphate)-1,3-(n-butylene)diamine-N-acetic acid;
N,N′-bis(pyridoxal-5-phosphate)-1,2-(3-methyl)propylenediamine-N-acetic acid;
N,N′-bis(pyridoxal-5-phosphate)-trans-1,2-cyclohexylenediamine-N-acetic acid;
N,N′bis(pyridoxal-5-phosphate)-trans-1,2-cyclopentylenediamine-N-acetic acid;
N,N′-bis(pyridoxal-5-phosphate)-trans-1,2-cycloheptylenediamine-N-acetic acid;
N,N′-bis(pyridoxal-5-phosphate)-trans-1,2-cyclooctylenediamine-N-acetic acid;
N,N′-bis(pyridoxal-5-phosphate)-1,2-phenylenediamine-N-acetic-acid;
N,N′-bis(pyridoxal-5-phosphate)-cis-1,2-cyclohexylenediamine-N-acetic acid;
N-pyridoxal-N′-(pyridoxal-5-phosphate)ethylenediamine-N,N′-diacetic acid (DPMP);
N,N′-bis(pyridoxal)-1,3-(n-propylene)-N,N′-diacetic acid;
N,N′-bis(pyridoxal)-1,2-(n-propylene)-N,N′-diacetic acid;
N,N′-bis(pyridoxal)-1,2-isopropylene-N,N′-diacetic acid;
N,N′-bis(pyridoxal)-1,2-(n-butylene)-N,N′-diacetic acid;
N,N′-bis(pyridoxal)-1,4-(n-butylene)-N,N′-diacetic acid N,N′-bis(pyridoxal-1,2-(3-methylene)propyl-N,N′-diacetic acid;
N,N′-bis(pyridoxal)-trans-1,2-cyclohexylenediamine-N,N′-diacetic acid;
N,N′-bis(pyridoxal)-trans-1,2-cyclopentylenediamine-N,N′-diacetic acid;
N,N′-bis(pyridoxal)-trans-1,2-cycloheptylenediamine-N,N′-diacetic acid;
N,N′-bis(pyridoxal)-trans-1,2-cyclooctylenediamine-N,N′-diacetic acid; and
N,N′-bis(pyridoxal)-1,2-phenylenediamine- N,N′-diacetic acid; and
N,N′-bis(pyridoxal)-cis-1,2-cyclohexylenediamine-N,N′-diacetic acid.

Particularly prferred chelating compounds of Formula I include:
N,N′-bis(pyridoxal-5-phosphate)ethylene-diamine-N,N′-diacetic acid (referred to hereinafter as DPDP); and
N,N′-bis(pyridoxal-5-phosphate)-trans-1,2-cyclohexyldiamine-N,N′-diacetic acid (referred to hereinafter as DPCP); and
N,N′-bis-(pyridoxal)-ethylenediamine-N,N-diacetic acid.

Another group of suitable chelating compounds include compounds of Formula II
$N(CH_2X)_3$ (II)
(wherein X represents a -COOY, $PO_3HY$ or -CONHOY group) and compounds of Formula III

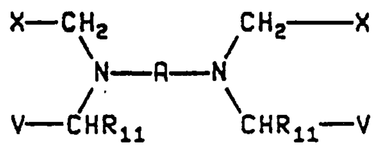

(III)

(wherein
X is as hereinbefore defined;
Y represents a hydrogen atom, a metal ion equivalent and/or a basic biocompatible cation of an inorganic or organic base or basic amino acid;
A represents a group
$-CHR_{12}-CHR_{13}-$,
$-CH_2CH_2(ZCH_2-CH_2)_m$,

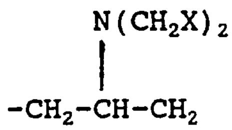

, or

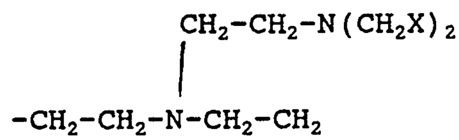

wherein X is as defined above;
each of the groups $R_{11}$ represents a hydrogen atom or a methyl group;
$R_{12}$ and $R_{13}$, which may be the same or different, each represents a hydrogen atom or a $C_{1-8}$ alkyl, phenyl or benzyl group, or
$R_{12}$ and $R_{13}$ together represent a $C_{1-8}$ alkylene group (e.g. trimethylene or tetramethylene etc);
m represents an integer from 1 to 3;
Z represents an oxygen or sulfur atom or a group

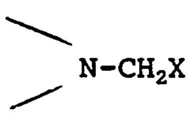

or

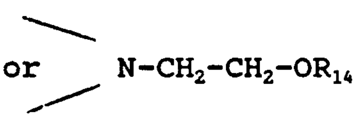

wherein X is as defined above;
$R_{14}$ represents a $C_{1-8}$ alkyl group;
Each of the groups V which may be the same or different represents a group X or a group
$-CH_2OH$ or $-CONH(CH_2)_nX$,
wherein X as defined above and
n represents an integer of from 1 to 12;
or if $R_{11}$, $R_{12}$ and $R_{13}$ represent hydrogen atoms, both the groups V together represent the group

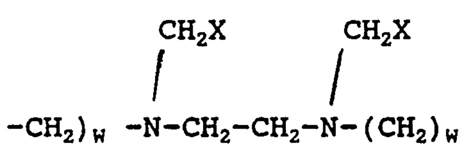

wherein X is as defined above, and W represents an integer of from 1 to 3).

By way of example suitable chelating compounds of Formula II include nitrolitriacetic acid and suitable

chelating compounds of Formula III include trans-1,2-cyclohexylenediamine-N,N,N′,N′-tetraacetic acid; 1,2-ethylenediamine-N,N,N′,N′-tetramethanesphosphonic acid; 1,4,7,10-tetraazacyclododecane-N,N′,N″,N‴-tetraacetic acid (DOTA); N,N′-bis(1-hydroxybenzyl)ethylenediamine-N,N′-diacetic acid; 13,23-dioxo-15,18,21-tris(carboxymethyl)-12,15,18,21,24-pentaaza-pentatriacontanedioic acid; 3,9-bis(1-carboxyethyl)-6-carboxymethyl-3,6,9-triazundecanedioic acid; ethylenediamine-N,N,N′,N′-tetraacetic acid (EDTA); ethylenedinitrilotetra(acetohydroxamic acid); 1,10-diaza-4,7-dithiadecane-1,1,10,10-tetraacetic acid; 1,2-diphenylethylenediaminetetraacetic acid; N′-(2-hydroxyethyl)ethylenediamine-N,N,N′-triacetic acid(HEDTA); 1,4,8,11-tetraazacyclotetradecane-N,N′,N″N‴-tetraacetic acid, 1,4,7,10-tetraazacyclododecane-N,N′,N″-triacetic acid; diisopropyl iminodiacetic acid; diethylenetrinitrilopenta(methylphosphonic acid); and 1-phenylethylenediaminetetracetic acid.

Especially preferred compounds of Formula II include diethylenetriaminepentaacetic acid (DTPA) and its analogs.

Compounds of Formula II and processes for their preparation are known and are described in US-A-4647447 and US-A-4639365, hereby incorporated by reference in their entirety.

A further group of suitable chelating compounds are those of Formula IV.

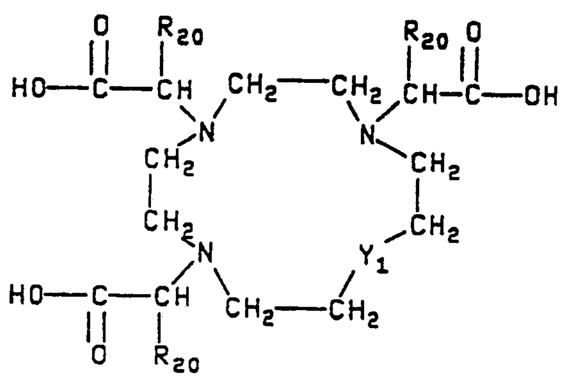

(IV)

(wherein,

$Y_1$ represents an oxygen or atom a group

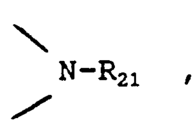

$R_{20}$ represents a hydrogen atom or an alkyl, arylalkyl or aryl group; and

$R_{21}$ represents a hydrogen atom or an alkyl, hydroxyalkyl or carboxyalkyl group), and physiologically tolerable salts thereof.

In Formula IV, alkyl moieties of $R_{20}$ or $R_{21}$ preferably contain 1 to 5 carbon atoms, and especially preferably are methyl and ethyl moieties are preferably optionally substituted phenyl, e.g., halo, alkyl, alkoxy, hydroxyl, carbamoyl or carboxyl substituted phenyl.

Preferred compounds of Formula III include those wherein $R_{20}$ is hydrogen, and $Y_1$ is carboxymethylimino (DOTA);

wherein $R_{20}$ is hydrogen, and $Y_1$ is alkylimino, preferably methylimino (DO3A);

4,7,10-triscarboxymethyl-1-oxo-4,7,10-triazacyclododecane(DOXA);

1,4,7-triscarboxymethyl-1,4,7,10-tetraazacyclododecane;

1-methyl-4,7,10-triscarboxymethyl-1,4,7,10-tetraazacyclododecane; and

1-benzyl-4,7,10-triscarboxymethyl-1,4,7,10-tetraazacyclododecane.

Chelating compounds of Formula III and methods for their preparation are described in EP-A-232751-(Squibb), the entire contents of which are hereby incorporated by reference. DOTA and its preparation are

described in US-A-4,647,447.

Especially preferred chelating compounds for use in accordance with the invention include DPDP, EDTP, EDTA, DTPA, DCTA, DOXA, DO3A, and DOTA.

Other chelating agents proposed in the literature for the preparation of paramagnetic metal chelates for use as MRI contrast media may of course be used. In this regard the reader is referred particularly to recent publications of Nycomed, Squibb, Bracco, Schering and Guerbet such as WO89/00557, EP-A-292689, EP-A-230893, EP-A-255471, EP-A-277088 and EP-A-287465. The entire disclosures of all of these documents are incorporated herein by reference.

Viewed from a further aspect, the present invention provides an MRI contrast medium comprising at least one pharmaceutically acceptable carrier or excipient together with a Mn(II) chelate according to the invention, or produced according to a process of the invention.

The contrast media of the present invention may be formulated with conventional pharmaceutical or veterinary formulation aids, for example stabilizers, antioxidants, osmolality adjusting agents, buffers, pH adjusting agents, etc., and may be in a form suitable for parenteral or enteral administration directly into a body cavity having an external escape duct, for example the gastrointestinal tract, the bladder or the uterus. Thus the contrast media of the present invention may be as tablets, capsules, powders, solutions, suspensions, dispersions, syrups, suppositories etc.; however, solutions, suspensions and dispersions in physiologically acceptable carrier media, for example water for injections, will generally be preferred.

The contrast media according to the invention may therefore be formulated for administration using physiologically acceptable carriers or excipients in a manner fully within the skill of the art. For example, the manganese chelate, optionally with the addition of pharmaceutically acceptable excipients, may be suspended or dissolved in an aqueous medium, with the resulting solution or suspension than being sterilized. As mentioned above, suitable additives include, for example, physiologically biocompatible buffers (as for example, tromethamine hydrochloride), slight additions of other chelating agents (as, for example, diethylenetriaminepentaacetic acid), or, optionally, calcium or sodium salts (for example, calcium chloride, calcium ascorbate, calcium gluconate or calcium lactate).

If the contrast media are to be formulated in suspension form, e.g. in water of physiological saline for oral administration, a small amount of an insoluble chelate salt may be mixed with one or more of the inactive ingredients conventionally present in oral solutions and/or surfactants and/or aromatics for flavouring.

The diagnostic media according to this invention conveniently contain from 0.001 to 5.0 moles per liter and preferably from 0.1 to 0.5 moles per liter of the manganese chelate.

The chelates of this invention are administered to patients for imaging in amounts which are sufficient to yield the desired contrast. Generally, dosages of from 0.001 to 5.0 mmoles of contrast agent per kilogram of patient bodyweight are effective to achieve reduction of relaxivity rates. The preferred dosages for most MRI applications are from 0.05 and 0.5 mmoles of contrast agent per kilogram of patient bodyweight.

Viewed from a still further aspect the invention provides the use of a substantially-protonated, water-insoluble Mn(II) chelate as defined herein for the preparation of contrast medium for use in diagnostic imaging, in particular MRI.

For MRI imaging of some portions of the body the most preferred mode for administering metal chelates as contrast agents is parenteral, e.g. intravenous administration. Parenterally administrable forms, e.g. intravenous solutions, should be sterile and free from physiologically unacceptable agents, and should have low osmolality to minimize irritation or other adverse effects upon administration and thus the contrast medium should preferably be isotonic or slightly hypertonic. Suitable vehicles include aqueous vehicles customarily used for administering parenteral solutions such as Sodium Chloride Injection, Ringer's Injection, Dextrose Injection, Dextrose and Sodium Chloride Injection, Lactated Ringer's Injection and other solutions such as are described in REMINGTON'S PHARMACEUTICAL SCIENCES, 15th ed., Easton: Mack Publishing Co, pp 1405-1412 and 1461-1487 (1975) and THE NATIONAL FORMULARY XIV, 14th ed. Washington: American Pharmaceutical Association (1975). The solutions can contain preservatives, antimicrobial agents, buffers and antioxidants conventionally used in parenteral solutions, excipients and other additives which are compatible with the chelates and which will not interfere with the manufacture, storage or use of products.

The contrast media of the invention may also, of course, be in concentrated or dried form for dilution prior to administration.

Viewed from a further aspect the invention provides a method of generating an image of a human or animal body, said method comprising administering to said body a contrast medium according to the invention and generating an image, e.g. an MR image, of at least part of said body, e.g., after permitting sufficient time to elapse for the medium to distribute to the desired parts of said body.

Methods for applying MRI contrast media to improve MR images, MRI equipment and MRI operating procedures are described by Valk et al., BASIC PRINCIPLES OF NUCLEAR MAGNETIC RESONANCE IMAGING, New York: Elsevier, PP 109-114 (1985).

This invention is further illustrated by the following non-limiting Examples. Temperatures are given in degrees centigrade and concentrations as weight percentages unless otherwise specified.

## EXAMPLE 1

### MnDPDP Precipitate

$H_8DPDP.2H_2O$ (1.00 g, 1.48 mmoles) and MnO (0.104 g, 1.46 mmoles) were slurried in 10 mL of water containing ascorbic acid (0.002 g, 0.01 mmoles) in a 20 mL erlenmeyer flask. The head space was purged with nitrogen, and the flask sealed with a rubber septum. Within 2 hours, the solution turned yellow, and no MnO particles were observed. The reaction was allowed to stir overnight. The yellow product was isolated by filtration and dried to a constant weight. A quantitative yield of product was obtained (MnDPDP, MW 691 g/mole).

The characterization follows:
Thermal Gravimetric Analysis (TGA): (30-270° C. 10°/min, $N_2$).
Calculation for trihydrate: 7.2%; found 8.2% (average of two runs).
Analysis calculated and (found) for
$C_{22}H_{30}MnN_4O_{14}P_2.3H_2O$: C, 35.44 (35.08); H, 4.83 (5.03); N, 7.52 (7.40); Mn, 7.37 (7.36)
Negative ion liquid secondary ion mass spectrometry (n-LSIMS) calculated for [M-1] peak and (found): m/z 690 (690).

## EXAMPLE 2

### MnEDTP Precipitate

$H_8EDTP$ (1.165 g, 2.50 mmoles) and MnO (0.176 g, 2.50 mmoles) were slurried in 10 mL of distilled water. The suspension was stirred and turned white after four hours. Solid $H_6MnEDTP$ (MW = 489.05 gm/mole) was isolated by filtration and washed with water and methanol before drying at 50° C. A quantitative yield of product was obtained.

The characterization follows:
Negative ion liquid secondary ion mass spectrometry (n-LSIMS) calculated and (found) for $[M-1]^-$ peak: m/z 488 (488).
Osmolality at pH 6.5 (normalized to 1 M): 3.26 Osmoles/kg.

## EXAMPLE 3

### MnEDTA Precipitate

$H_4EDTA$ (0.730 g, 2.50 mmoles) and MnO (0.176 g, 2.49 mmoles) were slurried in 10 mL of distilled water. The suspension was stirred and turned red in color after four hours. After stirring for an additional eight hours, the suspension had a slight pink color which turned to a completely white suspension upon heating at 45° C for ten minutes. Solid $H_2MnEDTA$ (MW = 345.17 gm/mole) was isolated by filtration and washed with water and methanol before drying at 50° C. A 50% yield of product was obtained.

The characterization follows:

Negative ion liquid secondary ion mass spectrometry (n-LSIMS) calculated and (found) for $[M-1]^-$ peak: m/z 344 (344).

Osmolality at pH 6.5 (normalized to 1 M); 2.54 Osmoles/kg.

## EXAMPLE 4

### MnDTPA Precipitate

$H_5DTPA$ (0.983 g, 2.50 mmoles) and MnO (0.177 gm, 2.50 mmoles) were slurried in 10 mL of distilled water. The suspension was stirred and turned green in colour after four hours. After stirring for an additional eight hours, the suspension turned white. Solid $H_3MnDTPA$ (MW = 446.28 gm/mole) was isolated by filtration and washed with water and methanol before drying at 50° C. A 50% yield of product was obtained.

The characterization follows:

Negative ion liquid secondary ion mass spectrometry (n-LSIMS) calculated and (found) for $[M-1]^-$ peak: M/z 445 (445).

Osmolality at pH 6.5 (normalized to 1 M): 2.98 Osmoles/kg.

## EXAMPLE 5

### MnDCTA Precipitate

$H_4DCTA$ (0.911 g, 2.50 mmoles) and MnO (0.176 g, 2.48 mmoles) were slurried in 10 mL of distilled water. The suspension was stirred and turned red in color after four hours. After stirring for an additional eight hours, the suspension had a slight pink color which turned to a completely white suspension upon heating at 45° C for ten minutes. Solid $H_2MnDCTA$ (MW = 399.26 gm/mole) was isolated by filtration and washed with water and methanol before drying at 50° C. A 25% yield of product was obtained.

The characterization follows:

Negative ion liquid secondary ion mass spectrometry (n-LSIMS) calculated and (found) for $[M-1]^-$ peak: m/z 398 (398).

Osmolality at pH 6.5 (normalized to 1 M): 2.56 Osmoles/kg.

## EXAMPLE 6

### Low Osmolarity Solution of MnDCTA

$H_4DCTA \cdot H_2O$ (18.2 g, 50.0 mmole) was slurried in $H_2O$ (50 mL) and solid NaOH (4.0 g, 100 mmole) was added with stirring. Green, insoluble MnO was added, and the slurry was slowly heated to 45° C for 30 min. The resultant clear solution of $MnNa_2DCTA$ was filtered through a 0.22 micron sterile filter into a septum vial which was sealed immediately.

The characterization follows:

Relaxivity:

$R_1$ (relaxation rate for $T_1$) for MnO-derived MnDCTA 3.60 $(mM\ sec)^{-1}$

$R_1$ for $MnCl_2$-derived MnDCTA 3.69 $(mM\ sec)^{-1}$

Acute Toxicity:

For MnO-derived MnDCTA: $LD_{50}$ (iv, Swiss-Webster mice), 5.4 mmole/kg

For $MnCl_2$-derived MnDCTA: $LC_{50}$ (iv, Swiss-Webster mice), 4.9 mmole/kg

## EXAMPLE 7

### Low Osmolarity Solution of MnDPDP

$H_8DPDP \cdot 2H_2O$ (9.76 g, 14.47 mmole) was slurried in $H_2O$ (20 mL) and solid NaOH (1.74 g, 43.4 mmole, 3.0 mole equivalents) was added with stirring. The clear, pale yellow solution was purged for 10 minutes with nitrogen. Green, insoluble MnO was added, and the slurry was stirred vigorously. After two hours, the homogenous yellow-orange solution was diluted volumetrically to 50 mL yielding a 289.3 mM solution. The $Na_3MnDPDP$ solution was filtered through a 0.22 micron sterile filter into a septum vial which was sealed immediately.

The characterization follows:

Osmolarity:

960 mOsm/KG at 289.3 mM = 3.3 Osmoles/kg at 1 M (theoretical is 4 Osmoles) for the above prepared solution. Osmolarity for solutions prepared from $MnCl_2$ = 7.6 Osmoles at 1 M (theoretical is 8 Osmoles).

Acute Toxicity:

For MnO-derived MnDPDP: $LD_{50}$ (iv, Swiss-Webster mice), 3.1 mmole/kg

For $MnCl_2$-derived MnDPDP: $LD_{50}$ (iv, Swiss-Webster mice), 2.5 mmole/kg

## EXAMPLE 8

### Osmolarity Values

The osmolarity values for 1 M solutions prepared with MnO compared to those prepared with $MnCl_2$ are summarized as follows:

| Compound | Prepared From | | | |
|---|---|---|---|---|
| | $MnCl_2$ | | MnO | |
| | Theory[a] | Found | Theory | Found |
| $MnDPDPH_xNa_{3-x}$ | 6-8 | 7.6 | 2-4 | 3.3 |
| $MnDCTAH_xNa_{2-x}$ | 6-7 | - | 2-3 | 2.6 |
| $MnEDTAH_xNa_{2-x}$ | 6-7 | - | 2-3 | 2.5 |
| $MnDTPAH_xNa_{3-x}$ | 7-8 | - | 3-7 | 3.0 |
| $MnEDTPH_xNa_{6-x}$ | 7-11 | - | 3-7 | 3.3 |

[a] Actual values depend upon solution pH.

## Claims

1. A process for the preparation of a Mn(II) chelate, said process comprising reacting MnO with a substantially protonated substantially water-insoluble chelating compound in an aqueous medium and optionally subsequently dissolving precipitated Mn(II) chelate in an aqueous base.

2. A process as claimed in claim 1 wherein said chelating compound has acidic protons.

3. A process as claimed in claim 1 or claim 2 wherein the reaction is carried out at a temperature of

from 20 to 50° C, and the MnO is reacted with at least one molar equivalent of the chelating compound.

4. A process as claimed in any one of claims 1 to 3 wherein the reaction is carried out at a pH of no more than 5.

5. A process as claimed in any one of claims 1 to 4 comprising separating precipitated Mn(II) chelate and dissolving said precipitate in a physiologically tolerable aqueous base solution.

6. A process for the preparation of an aqueous solution of a Mn(II) chelate, said process comprising reacting MnO with a chelating compound other than EDTA in a physiologically acceptable basic aqueous medium.

7. A process as claimed in claim 6 wherein said basic medium contains a molar equivalent or excess of a physiologically acceptable base.

8. A process as claimed in claim 7 wherein said reaction is carried out in the presence of from 1 to 6 molar equivalents of said base.

9. A process as claimed in any one of claims 6 to 8 wherein said base in aqueous solution yields cations selected from lithium, sodium, potassium and calcium and cations of ethanolamine, diethanolamine, morpholine, glucamine, N,N-dimethylglucamine, N-methyl glucamine, lysine, arginine and ornithine.

10. A process as claimed in any one of claims 1 to 9 wherein said chelating compound is a chelating compound of Formula I

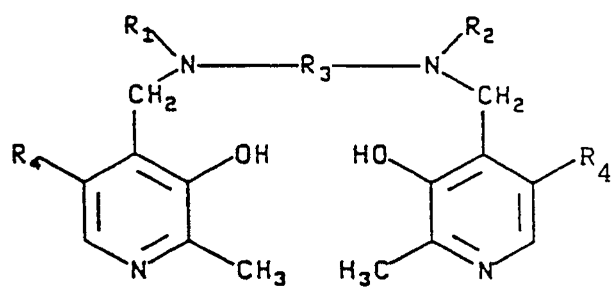

(I)

(wherein

$R_1$ represents a hydrogen atom or a group

$-CH_2-\overset{\overset{O}{\|}}{C}-R_5$, and

$R_2$ represents a hydrogen atom or a group

$-CH_2-\overset{\overset{O}{\|}}{C}-R_6$

with the proviso that one of the groups $R_1$ and $R_2$ is other than hydrogen;

$R_3$ represents a $C_{1-8}$ alkylene, $C_{5-8}$ 1,2-cycloalkylene, or $C_{6-10}$ 1,2-arylene group;

each of the groups $R_4$ which may be the same or different represents a hydrogen atom, a $C_{1-6}$ alkyl group or a group $-CH_2OH$, or a group

$-CH_2-O-\overset{\overset{O}{\|}}{P}(OH)_2$;

with the proviso that where one $R_4$ represents hydrogen or alkyl the other represents a group $-CH_2OP(O)(OH)_2$; $R_5$ and $R_6$ which may be the same or different each represents a hydroxy, $C_{1-18}$, optionally hydroxy-substituted, alkoxy, amino or $C_{1-8}$ alkylamino group),

and pharmaceutically acceptable salts, and where group $R_4$ represents a group $CH_2-O-PO(OH)_2$, the phosphate group mono and diesters thereof with mono and polyhydric $C_{1-18}$ alkyl- or alkylamino alcohols.

11. A process as claimed in any one of claims 1 to 9 wherein said chelating compound is a chelating compound of Formula II

$N(CH_2X)_3$ . . (II)

(wherein X represents a -COOY, $PO_3HY$ or -CONHOY group), or a physiologically tolerable salt thereof.

12. A process as claimed in any one of claims 1 to 9 wherein said chelating compound is a chelating compound of Formula III

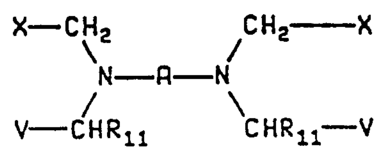

(III)

(wherein
X is as hereinbefore defined;
Y represents a hydrogen atom, a metal ion equivalent and/or a basic biocompatible cation of an inorganic or organic base or basic amino acid;
A represents a group
$-CHR_{12}-CHR_{13}-$,
$-CH_2CH_2(ZCH_2-CH_2)_M$,

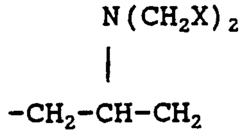

, or

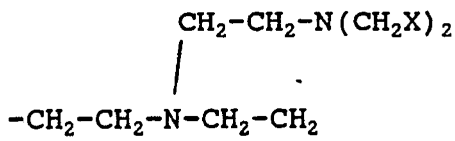

wherein X is as defined above;
each of the groups $R_{11}$ represents a hydrogen atom or a methyl group;
$R_{12}$ and $R_{13}$, which may be the same or different, each represents a hydrogen atom or a $C_{1-8}$ alkyl, phenyl or benzyl group, or
$R_{12}$ and $R_{13}$ together represent a $C_{1-8}$ alkylene group
m represents an integer, from 1 to 3;
Z represents an oxygen or sulfur atom or a group

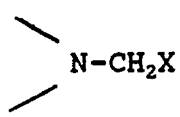

or

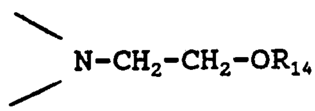

wherein X is as defined above;
$R_{14}$ represents a $C_{1-8}$ alkyl group;
Each of the groups V which may be the same or different represents a group X or a group $-CH_2OH$ or $-CONH(CH_2)_nX$,
wherein X as defined above and
n represents an integer of from 1 to 12;
or if $R_{11}$, $R_{12}$ and $R_{13}$ represent hydrogen atoms, both the groups V together represent the group

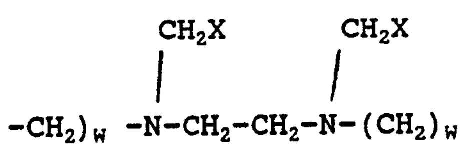

wherein X is as defined above, and W represents an integer of from 1 to 3),
or a physiologically tolerable salt thereof:

13. A process as claimed in any one of claims 1 to 9 wherein said chelating compound is a chelating compound of Formula IV

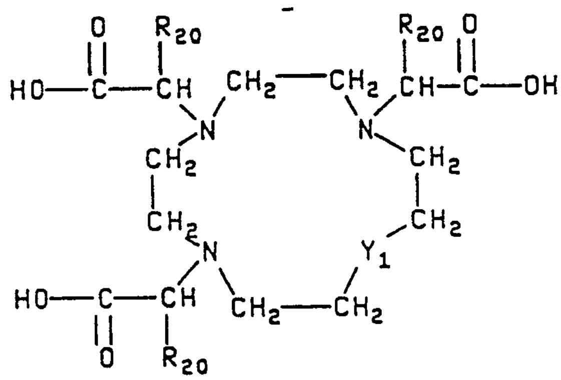

(IV)

(wherein
$Y_1$ represents an oxygen or atom a group

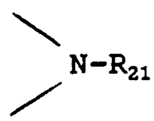

,

$R_{20}$ represents a hydrogen atom or an alkyl, arylalkyl or aryl group; and
$R_{21}$ represents a hydrogen atom or an alkyl, hydroxyalkyl or carboxyalkyl group), or a physiologically tolerable salt thereof.

14. A process as claimed in any one of claims 1 to 9 wherein said chelating compound is a chelating compound selected from DPDP, DTPA, DCTA, EDTP, DOXA, DO3A, DOTA and EDTA

15. A substantially-protonated, water-insoluble Mn(II) chelate.

16. A chelate as claimed in claim 15 being a chelate of Mn(II) with a chelating compound as defined in any one of claims 10 to 13.

17. A chelate as claimed in claim 15 being a chelate of Mn(II) with a chelating compound selected from DPDP, DTPA, DCTA, EDTP, DOXA, DO3A, DOTA and EDTA.

18. A magnetic resonance imaging contrast medium comprising at least one physiologically tolerable carrier or excipient together with a Mn(II) chelate as defined in any one of claims 15 to 17 or as prepared by a process as claimed in any one of claims 1 to 14.

19. Use of a substantially-protonated water-insoluble Mn(II) chelate as claimed in any one of claims 15 to 17 for the preparation of a contrast medium for use in diagnostic imaging.

20. A method of generating an image of a human or animal body, said method comprising administering to said body a contrast medium as claimed in claim 18, and generating an MR image of at least part of said body.

European Patent Office

# EUROPEAN SEARCH REPORT

Application Number

EP 89 20 2414

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 255 471 (SCHERING) * Example 18; claims * --- | 1,2,4-9,18,20 | C 07 F 9/58<br>C 07 F 9/38<br>C 07 C 229/16<br>A 61 K 49/00<br>C 07 D 257/02<br>C 07 D 273/00 |
| X | US-A-4 322 361 (WILSON et al.) * Example 12; claims * --- | 1,2,12 | |
| X | EP-A-0 250 358 (SCHERING) * Example 18; claims * --- | 1,2,4-9,15,18,20 | |
| A | DE-A-3 427 980 (B.A.S.F.) * Examples; claims * --- | 1,2,11,12,14 | |
| A | US-A-4 181 672 (POPPER et al.) * Examples 5-7,10; claims * --- | 1,2,11,12,14 | |
| A | DE-A-3 401 052 (SCHERING) * Examples 9,13,19,20,29,45,50,52; claims 1,2,6,14,16,18,21,38,42,44,48 * --- | 1,2,5-9,12,14 | |
| D,A | EP-A-0 232 751 (E.R. SQUIBB & SONS) * Example 10; claims * --- | 1,2,13,14,18,20 | **TECHNICAL FIELDS SEARCHED (Int. Cl.5)** |
| D,A | EP-A-0 292 761 (SALUTAR) * Whole document * ----- | 1,2,10,18,20 | C 07 C 229/00<br>C 07 F 9/00<br>C 07 D 257/00<br>C 07 D 273/00<br>C 07 F 13/00 |

The present search report has been drawn up for all claims

| Place of search | Date of completion of the search | Examiner |
|---|---|---|
| THE HAGUE | 06-02-1990 | HELPS I.M. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)